# EUROPEAN PATENT APPLICATION

(11) **EP 2 174 619 A1**
(43) Date of publication of application: **14.04.2010**
(21) Application number: 08791658.1
(22) Date of filing: 25.07.2008
(51) Int. Cl.: A61F 2/06, A61L 27/00

(54) **PROSTHETIC MATERIAL FOR LIVING ORGAN**

(30) Priority: 09.08.2007 JP 2007208467
(71) Applicant: Gunze Limited, Ayabe-shi, Kyoto 623-8511 (JP)
(72) Inventor: SAKAMOTO, Yuki, Ayabe-shi Kyoto 623-8512 (JP); MATSUDA, Shojiro, Ayabe-shi Kyoto 623-8512 (JP); SHINOKA, Toshiharu, Tokyo 156-0051 (JP); ICHIHARA, Yuki, Tokyo 132-0001 (JP); IKADA, Yoshito, Uji-shi Kyoto 611-0011 (JP)
(74) Representative: Albrecht, Thomas
(86) International application number: PCT/JP2008/063414
(87) International publication number: WO 2009/019995

(57) **Abstract**

It is an object of the invention to provide a prosthetic material for living organs, which can sufficiently withstand the arterial pressure and thereby can prevent leakage of blood or rupture of blood vessels, and which serves as a scaffold for vascular tissue regeneration, and does not necessitate repeated surgeries or the like.

The invention is a prosthetic material for living organs, which comprises a laminate including a film layer and a porous layer, said film layer comprising a bioabsorbable material, said porous layer comprising a bioabsorbable material.

## Description

### TECHNICAL FIELD

The present invention relates to a prosthetic material for use in living organs, which can sufficiently withstand the arterial pressure and thereby can prevent leakage of blood or rupture of blood vessels, and which serves as a scaffold for vascular tissue regeneration, and does not necessitate repeated surgeries or the like.

### BACKGROUND ART

Left ventricular hypoplasia syndrome is a disease in which blood vessels spanning from the left ventricle to the aortic valve and the aorta have been formed immaturely, and is a disease that currently exhibits the highest mortality rate in a pediatric cardiovascular surgery and faces difficulties in lifesaving. Furthermore, coarctation of aortic arch and interrupted aortic arch are diseases in which the middle of the aorta is severely constricted, or a portion of the aorta is interrupted. These congenital cardiac diseases in infants are all characterized by a deficiency of arterial blood vessels themselves. Thus, at present, a method of sacrificing other arteries of less importance compared with the aorta (for example, left subclavian artery or the like) and making an incision in the blood vessel to extend it (flap method), is utilized, or a technique of compensating for an absolute shortage of the vascular bed area with a patch material is carried out.

It has been a practice to use, for example, an autologous pericardium as such a patch material. The autologous pericardium has a feature of having high affinity to blood vessels and causing no problems such as rejection. However, since the amount of the autologous pericardium that can be used is limited due to its nature, and therefore, in fact, there has been a problem that the pericardium can be used only for the first surgery. Thus, an investigation is being made on prosthetic materials for living organs which are based on artificial materials.

As an example of the prosthetic materials for use in living organs, which are based on artificial materials, Patent Document 1 describes a ring-shaped vascular prosthetic material that includes an outer layer and an inner layer, which are non-bioabsorbable and porous and are formed from a polyester fiber or the like, and a non-bioabsorbable intermediate layer formed from silicone rubber or the like. Patent Document 2 describes a vascular prosthetic material, in which a porous inner layer formed from silicone rubber or the like, and an outer layer formed from a polyester fiber or the like are laminated. There has also been an attempt to utilize a so-called artificial blood vessel formed from polyethylene, a fluororesin or the like, as the prosthetic material for living organs.

However, when a prosthetic material for living organs that is formed from such a non-absorbable material, is used particularly in an infant, there are serious problems that the blood vessel at the part filled with the prosthetic material for living organs can not grow sufficiently along with the growth of the infant, and that the prosthetic material has a very high possibility of undergoing adhesion or calcification, and a reoperation is required for re-substitution.
Patent Document 1: WO 99/12496
Patent Document 2: Japanese Kokai Publication Hei-11-99163 (JP-A 11-99163)

### DISCLOSURE OF THE INVENTION

### PROBLEMS TO BE SOLVED BY THE INVENTION

In view of the above state of the art, it is an object of the present invention to provide a prosthetic material for living organs which can sufficiently withstand the arterial pressure and thereby can prevent leakage of blood or rupture of blood vessels, and which serves as a scaffold for vascular tissue regeneration, and does not necessitate repeated surgeries or the like.

### MEANS FOR SOLVING THE PROBLEMS

The present invention is a prosthetic material for living organs, which comprises a laminate including a film layer and a porous layer, said film layer comprising a bioabsorbable material, said porous layer comprising a bioabsorbable material.
Hereinafter, the present invention will be described in detail.

The present inventors devotedly conducted investigations, and as a result, they found that a laminate including a film layer containing a bioabsorbable material and a porous layer containing a bioabsorbable material can sufficiently withstand the arterial pressure and thereby can prevent leakage of blood or rupture of blood vessels when used as a prosthetic material for living organs, and at the same time, found that the laminate serves as a scaffold for vascular regeneration, thus making a blood vessel of a patient to be regenerated in an extremely short time, and also undergoes degradation and absorption after a certain time period so that calcification does not occur and it is not necessary to replace the prosthetic material by a reoperation. These findings have now led to completion of the present invention.

The prosthetic material for living organs of the present invention is formed from a laminate of a film layer and a porous layer.
The film layer is intended to impart to the prosthetic material for living organs of the present invention a strength that can sufficiently withstand the arterial pressure, so as to accomplish the role of preventing the leakage of blood. This film layer also accomplishes the roles of preventing the leakage of air, preventing to fall away of seeded cells, preventing the approach of tissues from one direction for a certain period of time (adhesion preventing effect), and the like.
The porous layer is constituted of a non-woven fabric, a sponge or a composite of these, and is intended to accomplish the role as a scaffold for vascular regeneration by seeding cells therein or allowing cells to invade from the surrounding blood vessels.

The bioabsorbable material that constitutes the film layer is not particularly limited, and examples thereof include synthetic absorbable polymers such as polyglycolide, polylactide (D-, L-, or DL-isomer), polycaprolactone, glycolide-lactide (D-, L-, or DL-isomer) copolymers, glycolide-ε-caprolactone copolymers, lactide (D-, L-, or DL-isomer)-ε-caprolactone copolymers, poly(p-dioxanone), and glycolide-lactide (D-, L-, or DL-isomer)-ε-caprolactone copolymers; and naturally occurring polymers such as gelatin, collagen and fibrin. These may be used individually, or two or more kinds thereof may be used in combination. Among them, at least one selected from the group consisting of lactide (D-, L-, or DL-isomer)-ε-caprolactone copolymers, glycolide-ε-caprolactone copolymers and glycolide-lactide (D-, L-, or DL-isomer) -ε-caprolactone copolymers is suitable, since they exhibit a high strength and flexibility, and an appropriate degradation behavior.

When the film layer is formed from a lactide (D-, L-, or DL-isomer)-ε-caprolactone copolymer or the like, a preferred lower limit of the content of the lactide in the lactide (D-, L-, or DL-isomer)-ε-caprolactone copolymer or the like is 40% by mole, and a preferred upper limit is 80% by mole. If the content of the lactide is less than 40% by mole, a strength required for withstanding the arterial pressure may not be obtained. If the content of the lactide exceeds 80% by mole, the flexibility required for adhering to a blood vessel may be insufficient. A more preferred lower limit of the content of the lactide is 50% by mole, and a more preferred upper limit is 75% by mole.

When the film layer is formed from a lactide (D-, L-, or DL-isomer)-ε-caprolactone copolymer or the like, a preferred lower limit of the weight average molecular weight of the lactide (D-, L-, or DL-isomer)-ε-caprolactone copolymer is 100000, and a preferred upper limit is 500000. If the weight average molecular weight of the lactide (D-, L-, or DL-isomer)-ε-caprolactone copolymer is less than 100000, a strength required for withstanding the arterial pressure may not be obtained. If the weight average molecular weight exceeds 500000, the solubility in a solvent may be decreased, or the processability may be decreased. A more preferred lower limit of the weight average molecular weight of the lactide (D-, L-, or DL-isomer)-ε-caprolactone copolymer is 150000, and a more preferred upper limit is 400000.

The thickness of the film layer is not particularly limited, but a preferred lower limit is 30 µm, and a preferred upper limit is 1 mm. If the thickness of the film layer is less than 30 µm, the film layer may not sufficiently withstand the arterial pressure and may undergo deformation or rupture. If the thickness exceeds 1 mm, the film layer may not be fixed to satisfactorily adhere to a blood vessel. Amore preferred lower limit of the thickness of the film layer is 50 µm, and a more preferred upper limit is 800 µm.

The bioabsorbable material that constitutes the porous layer is not particularly limited, and examples thereof include synthetic absorbable polymers such as polyglycolide, polylactide (D-, L-, or DL-isomer), glycolide-lactide (D-, L-, or DL-isomer) copolymers, glycolide-ε-caprolactone copolymers, lactide (D-, L-, or DL-isomer)-ε-caprolactone copolymers, poly(p-dioxanone), and glycolide-lactide (D-, L-, or DL-isomer)-ε-caprolactone copolymers. These may be used individually, or two or more kinds thereof may be used in combination. Among them, at least one selected from the group consisting of lactide (D-, L-, or DL-isomer)-ε-caprolactone copolymers, glycolide-ε-caprolactone copolymers and glycolide-lactide (D-, L-, or DL-isomer)-ε-caprolactone copolymers is suitable, since they exhibit a high strength and flexibility, and an appropriate degradation behavior.

When the porous layer is formed from a lactide (D-, L-, or DL-isomer)-ε-caprolactone copolymer or the like, a preferred lower limit of the content of the lactide in the lactide (D-, L-, or DL-isomer)-ε-caprolactone copolymer or the like is 40% by mole, and a preferred upper limit is 80% by mole. If the content of the lactide is less than 40% by mole, the strength may be insufficient. If the content of the lactide exceeds 80% by mole, the flexibility required for adhering to a blood vessel may be insufficient. A more preferred lower limit of the content of the lactide is 50% by mole, and a more preferred upper limit is 75% by mole.

When the porous layer is formed from a lactide (D-, L-, or DL-isomer) -ε-caprolactone copolymer or the like, a preferred lower limit of the weight average molecular weight of the lactide (D-, L-, or DL-isomer) -ε-caprolactone copolymer or the like is 100000, and a preferred upper limit is 700000. If the weight average molecular weight of the lactide (D-, L-, or DL-isomer)-ε-caprolactone copolymer or the like is less than 100000, a strength required for withstanding the arterial pressure may not be obtained. If the weight average molecular weight exceeds 700000, the solubility in a solvent may be decreased, or the processability may be decreased. A more preferred lower limit of the weight average molecular weight of the lactide (D-, L-, or DL-isomer) -ε-caprolactone copolymer or the like is 150000, and a more preferred upper limit is 500000.

The porous layer may be made of a non-woven fabric, a sponge, a composite of these, or the like. Among them, a non-woven fabric is suitable.
When the porous layer is made of a non-woven fabric, the mass per unit area is not particularly limited, but a preferred lower limit is 35 g/m², and a preferred upper limit is 300 g/m². If the mass per unit area of the porous layer is less than 35 g/m², even when cells are seeded or cells invade thereinto, the cell density may not be high, and it may be difficult for vascular regeneration to proceed. If the mass per unit area exceeds 300 g/m², invasion of cells into the inside of the porous layer becomes difficult, and the vascular regeneration may be interrupted. A more preferred lower limit of the mass per unit area of the porous layer is 50 g/m², and a more preferred upper limit is 250 g/m².
Likewise, when the porous layer is made of a sponge, or a composite of a sponge and a non-woven fabric, the pore size and the density are the same as in the case of the non-woven fabric described above.

The thickness of the porous layer is not particularly limited, but a preferred lower limit is 80 µm, and a preferred upper limit is 5 mm. If the thickness of the porous layer is less than 80 µm, the porous layer may not sufficiently function as a scaffold for vascular regeneration. If the thickness exceeds 5 mm, the film layer may not be fixed to satisfactorily adhere to a blood vessel. A more preferred lower limit of the thickness of the porous layer is 100 µm, and a more preferred upper limit is 4 mm.

When the porous layer is made of a non-woven fabric, the layer may be subjected to a hydrophilization treatment. If a hydrophilization treatment is applied, when the porous layer is contacted with a cell suspension, the porous layer can rapidly absorb this suspension, and cells can be seeded more efficiently and uniformly. Furthermore, the porous layer can be made more susceptible to the invasion of cells from the surrounding blood vessels.
The hydrophilization treatment is not particularly limited, and examples thereof include a plasma treatment, a glow discharge treatment, a corona discharge treatment, an ozone treatment, a surface grafting treatment, an ultraviolet irradiation treatment, and the like. Among them, a plasma treatment is suitable because the water absorption rate can be dramatically enhanced without changing the external appearance of the non-woven fabric.

The non-woven fabric may be integrated with a sponge formed from one of the bioabsorbable materials described above, one of polysaccharides such as starch, alginic acid, hyaluronic acid, chitin, pectinic acid and derivatives thereof, or one of naturally occurring polymers such as proteins including gelatin, collagen, albumin, and fibrin, and the like for the purpose of adjusting the size and the density of the pores and improving the adhesion of cells. As such, when a porous layer produced by integrating a sponge layer with a non-woven fabric to form a composite is used, particularly excellent cell invasiveness can be realized.

The prosthetic material for living organs of the present invention is preferably such that its bending stiffness, which is measured by a method equivalent to the cantilever test method stipulated in the bending stiffness A method of JIS L-1096 using a specimen having a size of 2 cm × 10 cm, is 10 cm or less. If the bending stiffness exceeds 10 cm, flexibility may be insufficient, and the prosthetic material may not be able to adhere to a blood vessel.

The method for producing the prosthetic material for living organs of the present invention is not particularly limited, but there may be mentioned, for example, a method of separately producing the film layer and the porous layer, subsequently dissolving a portion of the surface of the obtained film in an organic solvent, superimposing the porous layer thereon, and drying the assembly to integrate, and the like.
The method for producing the film layer is not particularly limited, and there may be mentioned, for example, a method of casting a solution prepared by dissolving the bioabsorbable material in an appropriate solvent, onto a releasable sheet, and then drying the solution, and the like.
The method for producing the porous layer (non-woven fabric) is not particularly limited, and there may be mentioned, for example, a needle punching method of producing a fiber of the bioabsorbable material by a melt spinning method or the like, weaving this fiber to obtain a fabric, and mechanically entangling the fiber of the fabric using a needle punching machine; a melt blowing method of simultaneously blowing out the bioabsorbable material that has been melted, through a number of nozzles to fabricate a fine fiber, concurrently disposing this fiber in all directions in a cobweb form to produce a web having a uniform thickness, and naturally or mechanically bonding the yarns; an ESD method of inducing a spray by applying a high voltage to the bioabsorbable material that has been melted, to produce a fine fiber, concurrently producing a web having a nanofiber size, uniformly on a substrate, and naturally or mechanically bonding the yarns; and the like.

The prosthetic material for living organs of the present invention is particularly useful as a patch material that is used in making an incision in the aorta at a constricted site to extend the aorta, and at the same time, compensating for the insufficient area of the blood vessel, in the treatment of congenital cardiac diseases in infants, such as the left ventricular hypoplasia syndrome, coarctation of aortic arch, and interrupted aortic arch. The prosthetic material can also be suitably used in the treatment of arteriosclerosis obliterans, true aortic aneurysm (dissociative aortic aneurysm), aortic dissection and the like.
In the prosthetic material for living organs of the present invention, cells crawl out of the surrounding blood vessels after implantation of the prosthetic material, to thereby invade into the porous layer. It can be also expected that vascular endothelial precursor cells and the like, which are present in the circulating blood, adhere to the porous layer, and either migrate or differentiate. Since the porous layer serves as a scaffold for vascular regeneration, an autologous blood vessel can be regenerated in a very early stage.
The prosthetic material for living organs of the present invention can also be suitably used in blood vessels, as well as in the urinary bladder (prevention of leakage of a body fluid), trachea (prevention of air leak), intestinal tract, cartilage, and the like.

The prosthetic material for living organs of the present invention can be used after a drug is made to soak into the porous layer.
The drug is not particularly limited, and examples thereof include aFGF, bFGF, EGF, VEGF, TGF-β, HGF, and the like as growth factors; and aspirin and the like as anti-thrombotic drugs. Examples also include heparin, warfarin, and the like as anti-coagulant drugs; cilostazol, aspirin, and the like as anti-platelet drugs; prednisolone, dexamethasone, cortisol, and the like as glucocorticoid drugs (steroid drugs) ; aspirin, diclofenac, indometacin, ibuprofen, naproxen, and the like as non-steroidal anti-inflammatory drugs (NSAIDs).

Furthermore, for the purpose of accelerating the regeneration of a blood vessel, endothelial cells, bone marrow cells, smooth muscle cells or fibroblast cells may be seeded in vitro into the porous layer before implantation, and the implantation may be carried out immediately without culturing the cells after seeding, or after culturing the cells in vitro. Alternatively, the prosthetic material may also be used directly without seeding cells in vitro.

A method for producing a prosthetic material for living organs for implantation, including seeding cells in vitro into the porous layer of the prosthetic material for living organs of the present invention, and further culturing the cells in vitro, is another aspect of the present invention.
A method for regenerating a tissue of the cardiovascular system, including regenerating a tissue of the cardiovascular system in vitro by seeding cells in vitro into the porous layer of the prosthetic material for living organs of the present invention, and further culturing the cells, is also another aspect of the present invention.
A prosthetic material for living organs for implantation that is obtainable by seeding endothelial cells, bone marrow cells, smooth muscle cells or fibroblast cells in vitro into the porous layer of the prosthetic material for living organs of the present invention, and further culturing the cells in vitro, is still another aspect of the present invention.

### EFFECT OF THE INVENTION

Accordingly, the present invention provides a prosthetic material for living organs, which can sufficiently withstand the arterial pressure and thereby can prevent leakage of blood or rupture of blood vessels, and which serves as a scaffold for vascular tissue regeneration, and does not necessitate repeated surgeries or the like.

### BEST MODES FOR CARRYING OUT THE INVENTION

Hereafter, the present invention will be described in detail by way of examples. However, the present invention is not intended to be limited only to these examples.

### (Example 1)

A fiber of an L-lactide-ε-caprolactone copolymer (molar ratio 75:25, weight average molecular weight 300000) was produced by a melt spinning method, and a fabric obtained by weaving this fiber was produced into a non-woven fabric using a needle punching machine. The thickness of the obtained non-woven fabric was about 3 mm.
Meanwhile, an L-lactide-ε-caprolactone copolymer (molar ratio 50:50, weight average molecular weight 200000) was dissolved in dioxane to thus prepare a 4 weight% dioxane solution. The obtained solution was flowed into a glass Petri dish and was subjected to air drying and heat treatment, and thereby a film having a thickness of about 100 µm was obtained.
The surface of one side of the obtained film was partially dissolved by applying a small amount of dioxane thereon, and the obtained non-woven fabric was laminated thereon. The laminate was dried to integrate the film and the non-woven fabric, and thus a prosthetic material for living organs was obtained. The thickness of the resulting prosthetic material for living organs was about 3 mm.

### (Example 2)

A fiber of polyglycolide (35 denier) was produced by a melt spinning method, and a fabric obtained by weaving this fiber was produced into a non-woven fabric using a needle punching machine. The thickness of the obtained non-woven fabric was about 700 µm.
Meanwhile, an L-lactide-ε-caprolactone copolymer (molar ratio 50:50, weight average molecular weight 200000) was dissolved in dioxane to thus prepare a 4 weight% dioxane solution.
The obtained non-woven fabric was interposed between glass plates, the obtained solution was flowed in between the glass plates, and then the assembly was frozen at -40°C.
Subsequently, the assembly was lyophilized and heat treated for 12 hours over a temperature range of -40°C to 40°C, and an assembly of a non-woven fabric integrated with a sponge was produced. The thickness of the resulting non-woven fabric/sponge was about 1 mm.

An L-lactide-ε-caprolactone copolymer (molar ratio 50:50, weight average molecular weight 200000) was dissolved in dioxane to thus prepare a 4 weight% dioxane solution. The obtained solution was flowed into a glass Petri dish and was subjected to air drying and heat treatment, and thereby a film having a thickness of about 100 µm was obtained.
The surface of one side of the obtained film was partially dissolved by applying a small amount of dioxane thereon, and the obtained non-woven fabric/sponge was laminated thereon. The laminate was dried to integrate the film and the non-woven fabric/sponge, and thus a prosthetic material for living organs was obtained. The thickness of the resulting prosthetic material for living organs was about 1 mm.

### (Evaluation by animal experiment)

An incision 30 mm long was made in the vertical direction in the descending aorta of each dog (beagle, body weight about 10 kg). The prosthetic material for living organs (20 mm × 30 mm) produced in Experimental Example 1 was placed at this incision, and the periphery was fixed by suture with a proline suture having a diameter of 6-0. After the surgery, there was not recognized any leakage of blood or rupture of the blood vessel from the part filled with the prosthetic material for living organs.
One, three and six months after the surgery, the dogs were sacrificed, and the blood vessels of the parts filled with the prosthetic material for living organs were extracted.
Photographs of the blood vessels of the parts filled with the prosthetic material for living organs shown in a vertically opened state, are presented in Fig. 1. Furthermore, tissue sections were produced, and microscopic photographs of hematoxylin eosin (HE) stain, Masson's trichrome (MS) stain, and Victoria blue (VB) stain in the vicinity of the sutured part are presented in Figs. 2 to 4, while a microscopic photograph of α-smooth muscle actin (αSMA) stain is presented in Fig. 5.

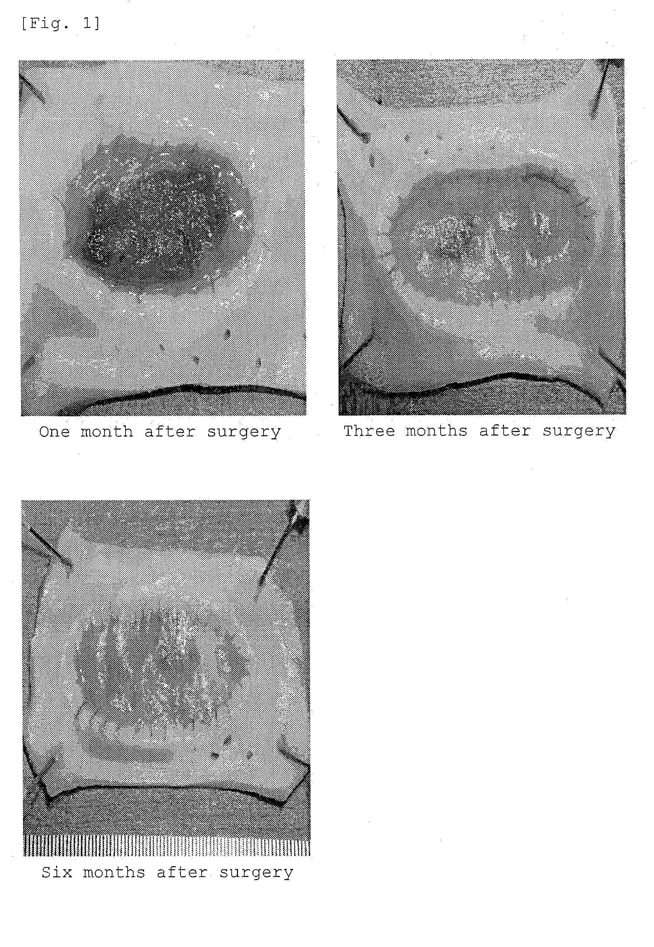

As can be seen from Fig. 1, although there is individual variability, one month after the surgery, the lumenal surface of the blood vessel at the part filled with the prosthetic material for living organs has already started endothelialization. The general view thereof seemed more mature after three months and six months, and thus tissues closer to the autologous arterial wall were formed.

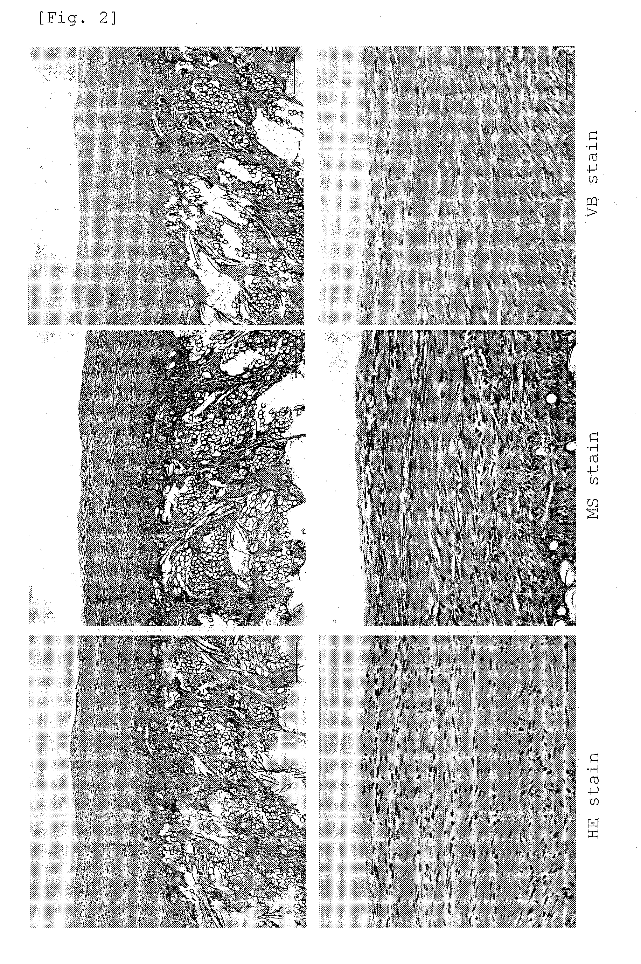

In Figs. 2 to 5, the upper side of the images represents the inner side of the blood vessel. Starting from one month after the surgery, proliferation of the tissue centering around the inner side of the blood vessel is recognized, but the invasion of cells into the interior of the non-woven fabric is low. After a lapse of three months from the surgery, the inner membrane tissue in the inner side of the blood vessel is thickened, while the thickness of the non-woven fabric is thinning. In six months after the surgery, the non-woven fabric as a scaffold has been mostly degraded and absorbed, but the vascular smooth muscle cells and the extracellular matrix centering around the collagen fiber further proliferated, to thereby form a more mature vascular wall structure.

### (Reference Example)

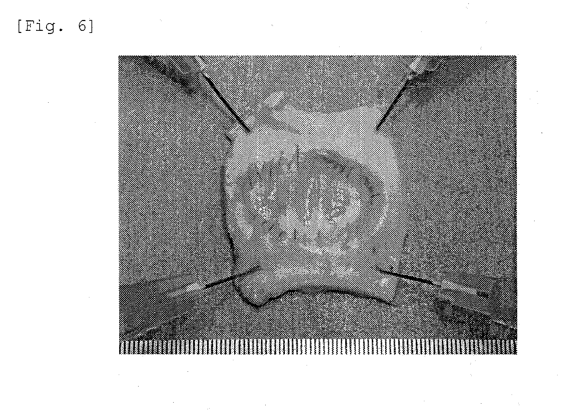

An incision 30 mm long was made in the vertical direction in the descending aorta of a dog (beagle, body weight about 10 kg) to extend the aorta. In order to compensate for this shortage of area of the blood vessel, a commercially available artificial blood vessel (trade name: Gelseal, a product produced by coating a fluororesin with gelatin) having a size of 20 mm × 30 mm was placed at this incision, and the periphery was fixed by suturing using a suture. After the surgery, there was not recognized any leakage of blood or rupture of the blood vessel from the part filled with the artificial blood vessel.
Twelve months after the surgery, the dog was sacrificed, and the blood vessel of the part filled with the artificial blood vessel was extracted. A photograph of the blood vessel of the part filled with the artificial blood vessel shown in a vertically opened state, is presented in Fig. 6.
It was found from Fig. 6 that in spite of the lapse of twelve months after the surgery, endothelialization scarcely proceeded.

### INDUSTRIAL APPLICABILITY

According to the present invention, there can be provided a prosthetic material for living organs, which can sufficiently withstand the arterial pressure and thereby can prevent leakage of blood or rupture of blood vessels, and which serves as a scaffold for vascular tissue regeneration, and does not necessitate repeated surgeries or the like.

### BRIEF DESCRIPTIONS OF THE DRAWINGS

Fig. 1 is a set of photographs showing the state of the blood vessel at the part filled with the prosthetic material for living organs of the present invention, obtained one, three and six months after the surgery.
Fig. 2 is a set of microscopic photographs of HE stain, MS stain and VB stain of the blood vessel at the part filled with the prosthetic material for living organs of the present invention, obtained one month after the surgery (upper row x50, lower row ×150).

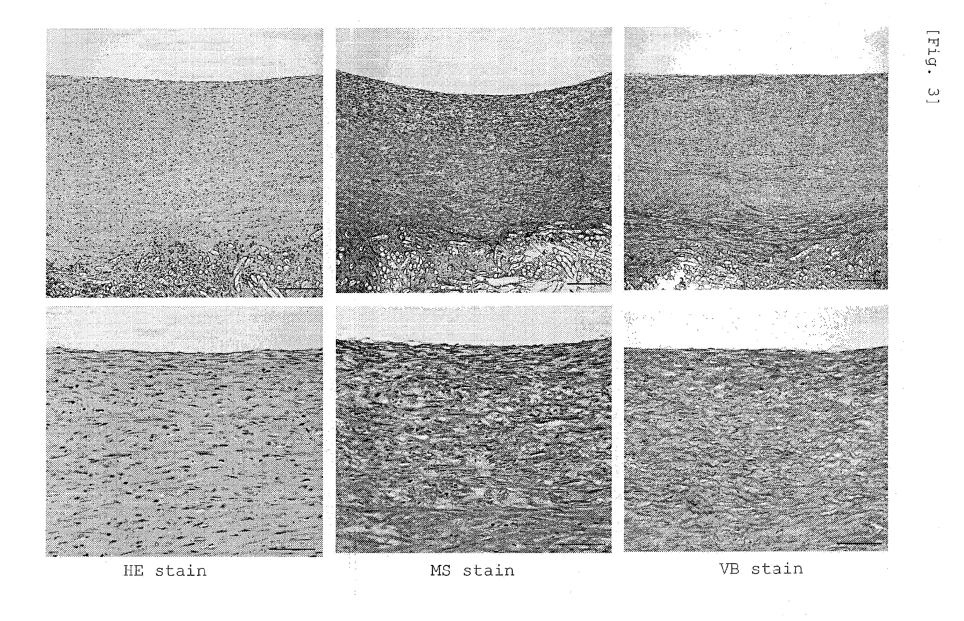

Fig. 3 is a set of microscopic photographs of HE stain, MS stain and VB stain of the blood vessel at the part filled with the prosthetic material for living organs of the present invention, obtained three months after the surgery (upper row x50, lower row ×150).

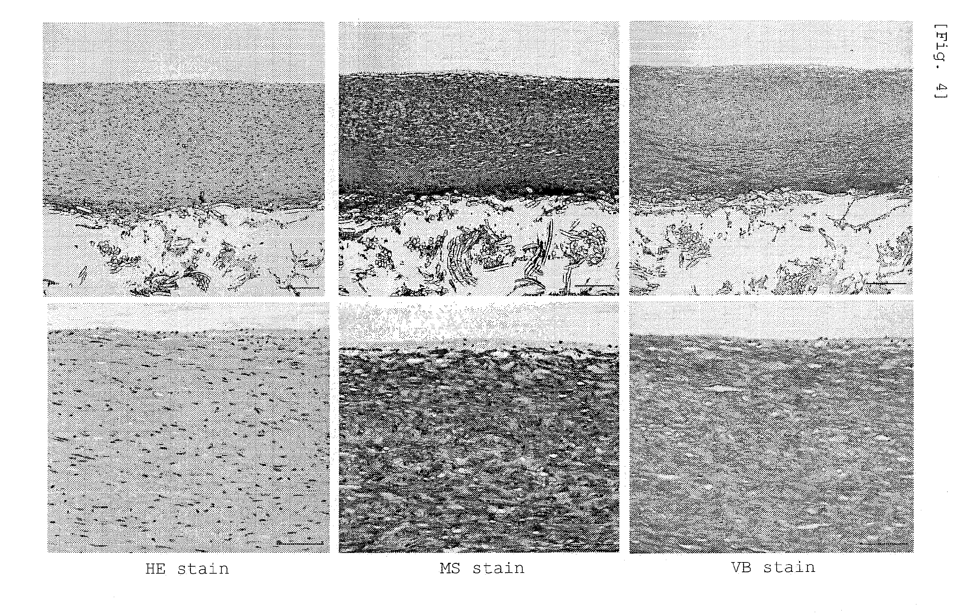

Fig. 4 is a set of microscopic photographs of HE stain, MS stain and VB stain of the blood vessel at the part filled with the prosthetic material for living organs of the present invention, obtained six months after the surgery (upper row x50, lower row ×150).

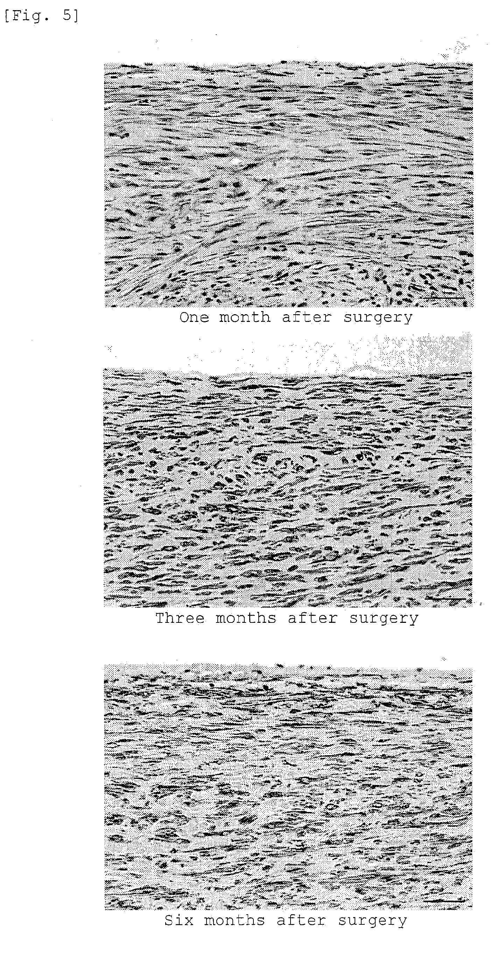

Fig. 5 is a set of microscopic photographs of αSMA stain of the blood vessel at the part filled with the prosthetic material for living organs of the present invention, obtained one, three and six months after the surgery (×200).
Fig. 6 is a photograph showing the state of the blood vessel at the part filled with a commercially available artificial blood vessel, obtained twelve months after the surgery.

## Claims

1. A prosthetic material for living organs,
which comprises a laminate including a film layer and a porous layer,
said film layer comprising a bioabsorbable material,
said porous layer comprising a bioabsorbable material.

2. The prosthetic material for living organs according to Claim 1,
wherein the bioabsorbable material constituting the film layer is a synthetic absorbable polymer or a naturally occurring polymer.

3. The prosthetic material for living organs according to Claim 1,
wherein the thickness of the film layer is in the range of 30 µm to 1 mm.

4. The prosthetic material for living organs according to Claim 2,
wherein the synthetic absorbable polymer is at least one selected from lactide (D-, L- or DL-isomer)-ε-caprolactone copolymers, glycolide-ε-caprolactone copolymers, and glycolide-lactide (D-, L-, or DL-isomer)-ε-caprolactone copolymers.

5. The prosthetic material for living organs according to Claim 2,
wherein the naturally occurring polymer is at least one selected from gelatin, collagen and fibrin.

6. The prosthetic material for living organs according to Claim 1,
wherein the porous layer is formed from a non-woven fabric, a sponge, or a composite of a non-woven fabric and a sponge.

7. The prosthetic material for living organs according to Claim 1,
wherein the bioabsorbable material constituting the porous layer is at least one selected from lactide (D-, L- or DL-isomer)-ε-caprolactone copolymers, glycolide-ε-caprolactone copolymers, and glycolide-lactide (D-, L-, or DL-isomer)-ε-caprolactone copolymers.

8. The prosthetic material for living organs according to Claim 6,
wherein the mass per unit area of the non-woven fabric layer is in the range of 35 to 300 g/m².

9. The prosthetic material for living organs according to Claim 1,
wherein the bending stiffness of the prosthetic material, which is measured by a method equivalent to the cantilever test method stipulated in the bending stiffness A method of JIS L-1096 using a specimen having a size of 2 cm × 10 cm, is 10 cm or less.

10. The prosthetic material for living organs according to Claim 1,
wherein a drug is soaked into the porous layer.

11. The prosthetic material for living organs according to Claim 1,
wherein cells are seeded in vitro in the porous layer.

12. The prosthetic material for living organs according to Claim 11,
wherein the seeded cells are bone marrow cells.
